# EUROPEAN PATENT SPECIFICATION

(11) **EP 0 479 354 B1**
(45) Date of publication and mention of the grant of the patent: **12.01.1994**
(21) Application number: 91202317.3
(22) Date of filing: 10.09.1991
(51) Int. Cl.: B01J 27/24, C01B 21/068

(54) **Nitrided silica**
Nitrierte Silica
Silice nitrurée

(30) Priority: 12.09.1990 GB 9019953
(43) Date of publication of application: 08.04.1992
(73) Proprietor: SHELL INTERNATIONALE RESEARCH MAATSCHAPPIJ B.V., 2596 HR Den Haag (NL)
(72) Inventor: Lednor, Peter William, NL-1031 CM Amsterdam (NL); de Ruiter, René, NL-1031 CM Amsterdam (NL); Sie, Swang Tiong, NL-1031 CM Amsterdam (NL)

(56) References cited:
- US-A- 4 053 513
- US-A- 4 888 159
- US-A- 4 985 224

## Description

The invention relates to a process for the preparation of nitrided silica particles and to the use of these silica particles as catalysts in the Knoevenagel reaction and other reactions.

In the U.S. Patent Specification 4,888,159 a process has been disclosed for the nitridation of silica wherein silica is converted with flowing ammonia-containing gas at temperatures of 950 °C to 1300 °C. Although the specification mentions that several kinds of silica may be converted with ammonia, it also discloses, however, that the active surface of silica gel is reduced by a factor 10.

It has now been found that nitrided silica particles may be prepared, which do not have the disadvantage of losing their active surface and do not collapse.

The invention relates to a process for the preparation of nitrided silica particles which comprises reacting porous silica particles of 0.5 mm diameter or more with an ammonia-containing gas at a temperature in the range of from 300 °C to 900 °C.

The silica particles, specifically the silica spheres of about 0.5 mm diameter or more, which are nitrided at their surface have modified catalytic properties in particular by (1) introduction of basicity and (2) suppression of residual acidity.

In the process according to the invention temperatures of 450 °C to 850 °C are preferably used.

Space velocities of flowing ammonia-containing gas are from 0.1 to 50 Nm³ per kg silica per hour.

The silica particles, to be nitrided, are preferably silica spheres, which do not collapse and for practical purposes retain much of their surface area. The outer surface is nitrided and can contain from about 0.5% by weight to about 5% by weight of N at the surface. Analysis by infrared spectroscopy showed that the nitrided silica contains ≡ Si-NH₂ groups and (≡ Si)₂-NH groups.

A process for the preparation of silica particles comprises:
a) preparing a silica hydrosol by mixing an aqueous solution of an alkali metal silicate with an aqueous solution of an acid,
b) converting the hydrosol into droplet form,
c) shaping the droplets in air or in a liquid which is not miscible with water, to obtain a hydrogel and
d) drying the hydrogel.

The process may be modified by decreasing the cation content by contact with an aqueous ammonium nitrate solution after step c). Another process consists of partially drying the hydrogel particles and subjecting them to a hydrothermal treatment at a temperature between 80 °C and 350 °C.

Optionally the dried hydrogels are calcined at a temperature up to 1000 °C, preferably between 800 °C and 950 °C.

The silica is generally known as silica gel, which latter is a partially dehydrated form of polymerized colloidal silica. As a finished, stable product, it is a hard granular material.

A more or less detailed procedure for the preparation of silica is given hereinafter.

The silica hydrosol has been conveniently prepared by mixing an aqueous solution of an alkali metal silicate with an aqueous solution of an acid. Suitable alkali metal silicates comprise the so-called waterglasses, based on Na₂O/SiO₂ having a sodium:silicon molar ratio between 1 and 0.2. Suitable acids comprise hydrochloric acid, nitric acid and especially sulphuric acid. Good results have been obtained using a molar ratio in the range 0.5-1.2 acid/waterglass, especially using a molar ratio in the range 0.6-0.8 acid/waterglass. The reactants can be used in various molar concentrations. Preferably, waterglasses are used in molar concentrations between 0.5 and 1.3 and the acid can be chosen accordingly.

The process of mixing has been performed suitably by leading the starting solutions separately via capillaries into tubular mixing chambers. Thorough mixing which appears to be important to produce uniform particles is established by carefully controlling the flow conditions in and outside the appropriate nozzles.

After the silica hydrosol has been formed, it is converted into droplet form by carefully controlling the break-up of the emerging stream which allows the formation of particles of uniform predetermined size and uniform shape. This may be achieved by gelling the hydrosol in a liquid which is immiscible, or substantially immiscible with water such as an oil, e.g. a paraffinic oil. This may be performed suitably by introducing the hydrosol into the upper end of a vertically disposed tube filled with oil. Best results are obtained when a relatively short sol-gel transformation time is applied, e.g. less than 15 seconds. It is also possible to spray the droplets in air.

The gelled particles thus obtained were caught in an aqueous phase such as water or, preferably, an aqueous solution of a salt such as sodium sulphate, particularly a salt solution having substantially the same salt concentration as that present in the hydrogel particles. The hydrogel particles are then separated from the aqueous phase, e.g. by filtration or centrifugation. The hydrogel particles thus obtained contain large amounts of water and also contain, apart from silica, water-soluble sodium salts as well as chemically bonded sodium ions.

It will be appreciated that silica particles of various sizes and shapes are used. Especially suitable are silica spheres which combine a number of advantageous properties such as high intrinsic strength, virtually no formation of fines when used in fixed or moving bed catalytic reactions as well as optimal transport characteristics.

Silica may also be prepared by partially pre-drying the hydrogel particles and a hydrothermal treatment of those particles.

Pure strong silica particles can be used when the amount of water present in the hydrogel is reduced very carefully, not only with respect to the temperature applied but also with respect to the amount of water which should remain in the then partially dried particles prior to the hydrothermal treatment.

The amount of water which is preferably removed from the hydrogel particles should preferably be between rather narrow ranges, e.g. between 45% by weight and 85% by weight, calculated on the amount of water initially present in the hydrogel. It has been found that very good results can be obtained when the amount of water remaining in the pre-dried particles is between 12% by weight and 70% by weight, especially between 30% by weight and 50% by weight, calculated on the total weight of the pre-dried particles.

Partial drying has been carried out conveniently by blowing air over the hydrogel particles either at constant temperatures or at slowly increasing temperatures provided that not even a small amount of the hydrogel particles are dried to completion. Temperatures ranging from ambient to 250 °C can be used, preference being given to temperatures in the range of from 70 °C to 180 °C.

When the hydrothermal treatment has been effected by treating the partially dried silica particles with liquid water at elevated temperature, in general a treating temperature between 50 °C and 374 °C is chosen. Preferred treating temperatures are between 80 °C and 350 °C and in particular between 100 °C and 300 °C. When using a treating temperature above 100 °C, the treatment has to be carried out in a closed vessel under autogenous pressure. The treating times generally range between 15 minutes and 24 hours. The volume of liquid water to be applied is preferably chosen such that during the treatment the partially-dried silica particles are completely surrounded by water.

Further details on hydrothermal treatment may be found in European Patent Specification 67459.

The hydrothermal treatment can be followed by a treatment to decrease the cation content of the silica particles to less than 10% by weight, preferably less than 5% by weight, calculated on dry material. Normally, the cations present are sodium ions originating from the waterglass constituent in the formation of the hydrosol. It is also possible that the cations are present since they were added to facilitate the hydrothermal treatment.

The decrease in the amount of cation was conveniently performed by washing the silica particles one or several times with water so that the concentration is reduced to the desired level. Also aqueous solutions of ammonium salts, e.g. ammonium nitrate have been used to reach the desired low alkali metal content of the silica particles. It should be noted that ammonium ions, which have replaced any cations bound to the silica particles will decompose during the subsequent drying and calcining treatment so that the final product does contain the desired amount of cations, if any.

According to the present invention the nitrided silica can be used as a starting material for the manufacture of shaped high-quality ceramic material and as a carrier material for catalytically functioning metal and as a catalyst.

The nitrided silica, prepared by the process according to the invention, displays an inert behaviour in respect to the double bond isomerization of butene-1 to butene-2. These products would therefore seem to be very suitable as carrier for a great number of reactions in the chemical process industry. Having no activity of its own can be very desirable in a catalyst carrier material. The nitrided silica may also be used as a basic catalyst, as shown by catalytic activity in the Knoevenagel condensation.

The nitrided silica may be used in a process for removing nitrogen oxides from a gaseous stream containing nitrogen oxides (NO, NO₂, NOₓ), wherein at elevated temperatures the gaseous stream is contacted with the nitrided silica particles. Suitably the temperature is selected between 100 °C and 500 °C. In this process nitrated silica donates NH₃ which reacts with NOₓ to form N₂ and H₂O.

The invention is further illustrated by the following examples.

### EXAMPLE 1

20 g of silica spheres having an average diameter of 1.5 mm, having a surface area of 318 m²/g, a pore volume of 1.66 ml/g and an average pore diameter of 34 nm were contained in a quartz reaction tube (inside diameter 35 mm) between two plugs of quartz wool. The quartz tube was placed in a horizontal tube oven.

The silica spheres were first treated to remove, by combustion, hydrocarbon contaminants. This treatment consisted of heating the spheres in a flow of pure oxygen (15 Nl/h) at 5 °C/min up to 500 °C, maintaining this temperature for two hours and allowing the spheres to cool to room temperature. The spheres were then flushed with nitrogen for one hour.

Nitridation of the spheres was carried out by heating them under an ammonia flow (30 Nl/h) at 5 °C/min up to desired temperature (either 500 °C or 800 °C), and maintaining the temperature for 4 h. After that the ammonia flow was replaced by a flow of dry nitrogen (15 Nl/h) and the spheres were allowed to cool to room temperature. X-ray photo-electron spectroscopy revealed that the silica, prepared at 500 °C and 800 °C, contained 0.8 and 1.2% by weight nitrogen in the surface, respectively. The surface area after nitridation at 800 °C was 276 m²/g.

### EXAMPLE 2

0.2 g of each of the silica spheres nitrided at 500 °C and 800 °C according to the process of Example 1 were suspended with stirring in 25 g of toluene at 50 °C.

0.424 g (0.4 mmol) of benzaldehyde and 0.456 g (0.4 mmol) of ethyl cyanoacetate were added to the suspension. The conversion was monitored by periodically taking samples for analysis by gas-liquid chromatography. After 4 h 58 and 81 percent of the reactants were converted respectively by using each of the silica samples. After 16 h 85 and 95 percent of the reactants were converted respectively by using each of the silica spheres.

### Comparative Example A

The same reaction of benzaldehyde with ethyl cyanoacetate was carried out under the same conditions as in Example 2, with the exception that the silica spheres were not nitrided. No conversion was measured.

### EXAMPLE 3

Determination of catalytic surface activity experiments were carried out with samples of the products prepared in Example 1, using butene-1 in helium, both with a flow rate of 5 litres per hour over 0.5 g of the samples to be tested. The outflowing gas was monitored by gas chromatography. Measurements were carried out at 400 °C, one hour after the catalyst had reached this temperature. Under these circumstances, the thermodynamic equilibrium quantity of butene-2 is 78%, silica gel (unnitrided) gave a conversion of 58% and the two nitrided silicas gave conversions of 14% and 13%, respectively. This demonstrates the less reactive nature of the nitrided silica surface.

## Claims

1. A process for the preparation of nitrided silica particles which comprises reacting porous silica particles of 0.5 mm diameter or more with an ammonia-containing gas at a temperature in the range of from 300 °C to 900 °C.

2. A process as claimed in claim 1 wherein the temperature lies in the range of from 450 °C to 850 °C.

3. A process as claimed in claim 1 or 2 wherein the ammonia-containing gas has a space velocity of 0.1 to 50 Nm³ ammonia/kg silica.h.

4. A process as claimed in claim 1 wherein silica spheres are used as starting materials.

5. A process as claimed in claim 4 wherein the silica is prepared by:
a) mixing an aqueous solution of an alkali metal silicate with an aqueous solution of an acid to obtain a silica hydrosol,
b) converting the hydrosol into droplet form,
c) shaping the droplets in air or in a liquid which is not miscible with water, to obtain a hydrogel,
d) and drying the hydrogel.

6. A process as claimed in claim 5 wherein the hydrogel particles are partially dried and subjected to a hydrothermal treatment at a temperature between 80 °C and 350 °C.

7. A process as claimed in claim 5 or 6 wherein the cation content of the hydrogels is decreased by treating them after step c) with an aqueous ammonium nitrate solution.

8. Use of nitrided silica particles, prepared by a process as claimed in one or more of the claims 1-7, in a process for removing nitrogen oxides from a gaseous stream containing said oxides, wherein at elevated temperatures the gaseous stream is contacted with the nitrided silica particles.

9. Use of nitrided silica prepared by a process as claimed in claims 1-7, as a catalyst in the Knoevenagel condensation of benzaldehyde with ethyl cyanoacetate.

10. Use of nitrided silica prepared by a process as claimed in claims 1-7, as a carrier material for catalytically functioning metal.

## Patentansprüche

1. Ein Verfahren zur Herstellung von nitrierten Siliziumoxidteilchen, welches das Zur-Reaktion-bringen von porösen Siliziumoxidteilchen mit einem Durchmesser von 0,5 mm oder mehr mit einem ammoniakhaltigen Gas bei einer Temperatur im Bereich von 300°C bis 900°C umfaßt.

2. Ein Verfahren wie in Anspruch 1 beansprucht, in dem die Temperatur im Bereich von 450°C bis 850°C liegt.

3. Ein Verfahren wie in Anspruch 1 oder 2 beansprucht, in dem das ammoniakhaltige Gas eine Raumgeschwindigkeit von 0,1 bis 50 Nm³ Ammoniak/kg Siliziumoxid.h aufweist.

4. Ein Verfahren wie in Anspruch 1 beansprucht, in dem Siliziumoxidkugeln als Ausgangsmaterial verwendet werden.

5. Ein Verfahren wie in Anspruch 4 beansprucht, in dem das Siliziumoxid hergestellt wird durch:
a) das Vermischen einer wässrigen Lösung eines Alkalimetallsilikats mit einer wässrigen Lösung einer Säure, um ein Siliziumoxidhydrosol zu erhalten,
b) das Umwandeln des Hydrosols in Tröpfchenform,
c) das Formen der Tröpfchen in Luft oder in einer Flüssigkeit, welche nicht mit Wasser vermischbar ist, um ein Hydrogel zu erhalten,
d) und das Trocknen des Hydrogels.

6. Ein Verfahren wie in Anspruch 5 beansprucht, in dem die Hydrogelteilchen teilweise getrocknet und einer hydrothermalen Behandlung bei einer Temperatur zwischen 80°C und 350°C unterworfen werden.

7. Ein Verfahren wie in Anspruch 5 oder 6 beansprucht, in dem der Kationgehalt der Hydrogele verringert wird, indem die Hydrogele Schritt c) mit einer wässrigen Ammoniumnitratlösung behandelt werden.

8. Die Verwendung von nitrierten Siliziumoxidteilchen, hergestellt durch ein Verfahren, wie in einem oder mehreren der Ansprüche 1 bis 7 beansprucht, in einem Verfahren zum Entfernen von Stickstoffoxiden aus einem gasförmigen Strom, welcher diese Oxide enthält, in dem bei erhöhten Temperaturen der gasförmige Strom mit den nitrierten Siliziumoxidteilchen in Berührung gebracht wird.

9. Die Verwendung von nitriertem Siliziumoxid, hergestellt durch ein Verfahren, wie in den Ansprüchen 1 bis 7 beansprucht, als ein Katalysator in der Knoevenagel-Kondensation von Benzaldehyd mit Ethylcyanoacetat.

10. Die Verwendung von nitriertem Siliziumoxid, hergestellt durch ein Verfahren, wie in den Ansprüchen 1 bis 7 beansprucht, als ein Trägermaterial für katalytisch wirkendes Metall.

## Revendications

1. Procédé de préparation de particules de silice nitrurée, qui consiste à faire réagir des particules de silice poreuse d'un diamètre de 0,5 mm ou plus avec un gaz contenant de l'ammoniac à une température de 300 à 900°C.

2. Procédé selon la revendication 1, dans lequel la température est comprise entre 450 et 850°C.

3. Procédé selon la revendication 1 ou 2, dans lequel le gaz contenant de l'ammoniac présente une vitesse spatiale de 0,1 à 50 Nm³ d'ammoniac/kg de silice.heure.

4. Procédé selon la revendication 1, dans lequel on utilise des sphères de silice comme matière de départ.

5. Procédé selon la revendication 4, dans lequel on prépare la silice comme suit :
(a) on mélange une solution aqueuse d'un silicate de métal alcalin avec une solution aqueuse d'un acide pour obtenir un hydrosol de silice,
(b) on convertit l'hydrosol en gouttelettes,
(c) on façonne les gouttelettes à l'air ou dans un liquide qui n'est pas miscible avec l'eau pour obtenir un hydrogel,
(d) et on sèche l'hydrogel.

6. Procédé selon la revendication 5, dans lequel on sèche partiellement les particules d'hydrogel et on les soumet à un traitement hydrothermique à une température de 80 à 350°C.

7. Procédé selon la revendication 5 ou 6, dans lequel on abaisse la teneur en cations des hydrogels en les traitant après le stade (c) par une solution aqueuse de nitrate d'ammonium.

8. Utilisation de particules de silice nitrurée préparées par un procédé selon l'une quelconque des revendications 1 à 7, dans le cadre d'un procédé pour éliminer les oxydes d'azote d'un courant gazeux qui contient lesdits oxydes, le courant gazeux étant mis en contact à une température élevée avec les particules de silice nitrurée.

9. Utilisation de la silice nitrurée préparée par un procédé selon les revendications 1 à 7, en qualité de catalyseur dans la condensation de Knoevenagel du benzaldéhyde avec le cyanoacétate d'éthyle.

10. Utilisation de la silice nitrurée préparée par un procédé selon les revendications 1 à 7 en qualité de support pour un métal à fonction catalytique.
